# EUROPEAN PATENT APPLICATION

(11) **EP 4 549 486 A1**
(43) Date of publication of application: **07.05.2025**
(21) Application number: 23207026.8
(22) Date of filing: 31.10.2023
(51) Int. Cl.: C08G 63/183, C08J 11/24

(54) **MONOMER RECYCLING OF POLYESTERS**

(71) Applicant: Polymetrix AG, 9245 Oberbüren (CH)
(72) Inventor: Christel, Andreas, 9524 Zuzwil (CH); Mattes, Kurt Michael, 9242 Oberuzwil (CH)
(74) Representative: Hepp Wenger Ryffel AG

(57) **Abstract**

The present invention is related to a process for the preparation of a polyester with a repeat unit [-A"-B"] having the formula [-O-R¹-OOC-R²-CO-], comprising the step of heating a recycled monomer product that is obtained from depolymerization of a polyester together with 3 - 50% by weight of water in a reactor to a temperature above the melting point of the condensation monomer at a pressure of at least 0.5 bar above ambient pressure, to obtain a modified recycled monomer product having an increase concentration of COOH end groups. The present invention is also related to the use of a polyester prepared from said modified recycled monomer product in a solid state polycondensation.

## Description

The present invention is related to a method for monomer recycling from a condensation polymer which is a polyester and the manufacture of a polyester from a recycled monomer product.

The preparation of polymers from their monomers is well known and established in large scale. Traditionally the used monomers are derived from fossil resources. The use of fossil resources is based on a linear product flow from fossil resource to polymer to waste. In the long term such linear product flows are not sustainable. As an alternative, the use of monomers derived from renewable resources is proposed to create a more cyclic product flow. However, the resulting cycle from polymer to CO₂ and water to plant based renewable resources to polymer is a long and energy intensive approach to provide polymers.

Especially condensation polymers have the advantage that they can be depolymerized into their monomers and re-polymerized from such recycled monomers. Depending on the depolymerization technology, the same monomers may be obtained that are typically used for the preparation of virgin polymers. In other cases, the recycled monomers may have a different composition and therefore are limited in their use in existing large-scale manufacturing processes. Such limiting differences may be the melting point of a solid monomer, the viscosity of a liquid monomer or the required energy to melt or evaporate the monomers. In particular, differences result from predefined mole ratios of reacted monomers.

Polyesters have a general structure comprising repeat units. They are typically prepared in large scale by polymerizing monomers with each other, wherein said monomers are diols and diacids.

Polyesters are typically prepared in large scale by polymerizing a diol monomer with a substituted diacid monomer. Alternatively, the polyesters can be prepared in large scale by polymerizing cyclic dimers, trimers or oligomers obtained from diol and diacid monomers. Due to ring opening polymerization, a polyester is formed.

These polyesters can be recycled by depolymerization with the addition of a diol monomer. The depolymerization results in a recycled monomer product comprising a condensation monomer having the formula HO-R¹-OOC-R²-COO-R¹-OH, wherein R¹ and R₂ are as defined below.

The depolymerization of polyesters with a diol monomer is well known and described for uses with post-industrial and post-consumer wastes. Especially when post-consumer wastes are involved, organic contaminants which might have migrated into the polyester or which might have been bound to the polyester molecule and subsequently been liberated during depolymerization have to be removed. Such removal is described by Welle: Safety Evaluation of Polyethylene Terephthalate Chemical Recycling Processes; Sustainability 2021, 13, 12854, 1-10. After depolymerization, various cleaning steps lead to the removal of organic contaminants. A typical cleaning step is based on crystallization and subsequent filtration. This leaves a monomer product with a significant amount of residual liquid, which has to be eliminated to obtain a pure condensation monomer.

Conventionally, these cleaning steps are carried out under conditions that prevent decomposition of the condensation monomer described above, which in the case of glycolysis of PET with ethylene glycol is bis-(β-hydroxyethyl) terephthalate (BHET).

For example, in US-3,668,235 (Teijin) a process for removing a volatile liquid medium from solid BHET wet with said volatile liquid medium and recovering dry BHET is described, which comprises heating and fusing solid BHET wet with a volatile liquid medium having a boiling point at normal pressure not lower than 40° C, but lower than the boiling point of ethylene glycol into a molten mass, and evaporating said volatile liquid medium from the melt without causing degradation of said BHET.

In WO 2022/180563 A1 (Garbo), a process for producing BHET in liquid form by depolymerization of polyethylene terephthalate (PET) is described, which comprises the depolymerization of PET with ethylene glycol, the purification and crystallization of the thus obtained BHET, and melting the thus obtained BHET in crystalline form (which is impregnated with a solvent) at a temperature lower than or equal to 90 °C and higher than or equal to 70 °C, to obtain a solution of BHET in said solvent. Said BHET solution is described as being very stable, with no oligomer formation and only a slight decrease in the BHET monomer content due to hydrolysis after 2 weeks of heating, which resulted in an increased acidity (formation of 2.1 mol-% carboxyl end groups). It is argued that higher acidity of BHET is an advantage from the point of view of PET production, as it increases time reactivity of BHET and thus the polycondensation rate. However, the amount of free carboxyl groups (CEG), measured according to ASTM D7409-15, should be from 40 to 100 mmol/kg (which is below 3 mol-%), more preferably from 60 to 80 mmol/kg. Thus, in WO 2022/180563 A1 it is merely suggested that the small amount of free carboxyl groups that is formed during the process described therein should not be detrimental. There is no suggestion that this amount could be significantly increased without any adverse influence on the properties of the desired product BHET.

From a BHET as described in the prior art discussed above, typically a polyester with a low content of COOH end groups is obtained. Such a polyester has the disadvantage that it can only undergo slower solid state polycondensation predominantly based on transesterification, resulting in diol monomer A as a condensation product.

It was the problem of the present invention to provide an improved process of preparing a polyester from a recycled monomer product.

The above problem is solved by the present invention.

In detail, the present invention is related to a process for the preparation of a polyester with a repeat unit [-A''-B''] having the formula [-O-R¹-OOC-R²-CO-], comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
b1) heating the recycled monomer product together with 3 - 50% by weight of water in a reactor to a temperature above the melting point of the condensation monomer at a pressure of at least 0.5 bar, but not more than 10 bar, above ambient pressure, and
b2) heating the modified recycled monomer product from step b1), preferably at a reduced pressure, to obtain a polyester polymer.

It has been found that a modified recycled monomer product obtained according to the present invention is more suitable for producing a polyester with higher intrinsic viscosity by solid state polymerization.

Recycled monomer product obtained by the steps of a1) to a3) of the process of the present invention predominantly comprises substances with OH end groups, while very few COOH end groups are present. Even if condensation monomers with one OH and one COOH end group, having the formula [HO-R¹-OOC-R²-COOH], remain or are formed after step a3) of the process of the present invention, the separation of the recycled monomer product in the preferred optional step a6) will reduce their content. Typically, a recycled monomer product recovered from depolymerization of a polyester with a diol monomer will have less than 10 mol-%, preferably less than 5 mol-%, more preferably less than 3 mol-% of COOH end groups.

According to the process of the present invention, by heating under pressure in step b1), this recycled monomer product is converted into a modified recycled monomer product, optionally containing low molecular weight reaction product thereof, which has a COOH end group concentration which is at least 9 mol-%, preferably at least 14 mol-%, more preferably at least 19 mol-% higher than the COOH end group concentration of the recycled monomer product.

If the converted monomer product is used for polymerization of a polyester without further addition of diol monomer A, then the COOH end group concentration must not exceed 50 mol-%.

The modified recycled monomer product is obtained by heating a recycled monomer product containing 50% to 97% by weight of a condensation monomer, having the formula HO-R¹-OOC-R²-COO-R¹-OH, and 3% to 50% by weight of water under pressure until it has sufficiently reacted to contain at least a 9% higher content of COOH end groups. Preferably, heating of the recycled monomer product together with the water is performed at a temperature above the melting point of the condensation monomer and at a pressure of at least 0.5 bar, but not more than 10 bar, above ambient pressure.

The present invention is also related to a modified recycled monomer product that is obtainable by the process of the present invention and comprises a blend of a condensation monomer having the formula HO-R¹-OOC-R²-COO-R¹-OH and of a modified condensation monomer having the formula [HO-R¹-OOC-R²-COOH], wherein R¹ and R² are as defined above, characterized in that said modified recycled monomer product has a COOH end group concentration in the range from 10 mol-% to 50 mol-%, preferably 15 mol-% to 45 mol-%, and especially preferred 20 mol-% to 40 mol-%.

The recycled monomer product provided to step b1) contains at least 50% and up to 100%, more preferably at least 70%, most preferably at least 80%, and preferably less than 95%, most preferred less than 90% by weight, on dry basis of a condensation monomer having the formula HO-R¹-OOC-R²-COO-R¹-OH, and 3% to 50%, preferably more than 5%, more preferably more than 10% and preferably less than 40% by weight of water, wherein the water content is based the total weight of recycled monomer product plus water. In a preferred embodiment of the present invention, the modified recycled monomer product contains at least 10 mol-%, more preferably at least 15 mol-%, most preferably 20 mol-% of a modified condensation monomer having a polyester repeat unit [-A"-B"] with an OH and a COOH end group, having the formula [HO-R¹-OOC-R²-COOH] .

Optionally the recycled monomer product may contain other substances, such as diol monomer A or dimers thereof or diacid B, dimers or low molecular weight oligomers of the condensation monomer or comonomers, which differ in R¹ or R² from the main condensation monomer.

In step b1), the recycled monomer product is heated to a temperature above the melting point of the condensation monomer, preferably to above 120°C, more preferably to above 135°C. Heating to the reaction temperature may be achieved by a separate heat exchanger and/or a heated reaction vessel.

As a result of said heating in step b1), the pressure increases. According to the present invention, heating results in a pressure of at least 0.5 bar, preferably at least 1.2 bar, more preferably at least 2.0 bar, but not more than 10 bar, preferably not more than 7 bar, above ambient pressure. To maintain the pressure within the desired range, excess gas phase may be released. A temperature ramp may be employed to maintain reaction conditions for a longer time before excess gas phase is removed.

The reaction time to prepare the modified recycled monomer product in step b1) depends on the initial water content and the reaction temperature. It is determined according to the above requirements. Typically, the reaction time is in the range of 5 minutes to 10 hours, preferably below 4 hours. Conditions must be sufficient to promote the desired reaction, causing the water to react with the condensation monomer or a low molecular weight reaction product thereof.

As a result of the conversion reaction in step b1), diol monomer A will be formed. A part of the diol monomer A will also be in the gas phase, allowing the removal of the monomer. This allows to recover diol monomer A at the site of recycling and reduces the need to remove excessive amounts of diol monomer A in polymer manufacturing.

In step b2), the thus obtained modified recycled monomer product is heated at a reduced pressure to obtain a polyester polymer, as will be discussed below.

According to the present invention, it has been found that the polyester obtained after step b2) can be used as a starting polyester for solid state polycondensation (SSP), wherein in said solid state polycondensation a high viscosity polyester with at least 0.1 dl/g higher IV than the starting polyester is prepared, and the solid state polycondensation reaction is based on esterification and transesterification, where the ratio of esterification to the total of esterification and transesterification is higher than 25%, preferably higher than 30%, more preferably more than 35%, and in some cases even above 50%.

The determination of the ratio of the above reactions is done by conventional methods, such as a calculation from measured values of intrinsic viscosity and COOH end groups, or by measurement of COOH and OH end groups.

Polyesters obtained from the above described methods are essentially free of contaminants and suitable to prepare packaging materials for direct food contact, such as bottles, jars, trays or film, even if the waste stream originates from non-food applications.

It is a further advantage of the present invention that said increase of the concentration of COOH end groups, which is the cause of the improvement observed in the subsequent SSP reaction, can be achieved without any addition of virgin acid monomer, such as TPA. Accordingly, the present invention provides for an increased recycling performance.

In the process of the present invention, any bisphenol A (BPA) that may be present in the recycled monomer product and/or the modified recycled monomer product is inserted as a building block into the final polyester obtained after step b2). Presence of BPA in the recycled monomer product and/or the modified recycled monomer product is not detrimental.

The following designations will be used herein for the condensation polymers, their depolymerization products and their monomers.

**Monomer:** A general term for building blocks of polymers. For condensation polymers, monomers comprise individual monomers, substituted monomers and condensation monomers.

**Individual monomer:** The basic building blocks of the polymer in unreacted form. According to the present invention, monomer A is a diol having the formula HO-R¹-OH and monomer B is a diacid having the general formula HOOC-R²-COOH, wherein R¹ and R² throughout this specification are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms or heterocyclic rings containing 1 to 3 oxygen atoms and 3 to 10 carbon atoms.

**Substituted monomer:** A monomer C obtained as a condensation product of individual monomer B HOOC-R²-COOH with two terminating molecules R³-OH, which will not form a part of the repeat unit of the related condensation polymer, i.e. the terminating molecules will be eliminated during the polycondensation reaction which results in a condensation polymer, and wherein R³ are aliphatic hydrocarbons containing 1 to 15 carbon atoms. According to the present invention, substituted monomer C has the formula R³OOC-R²-COOR₃. In one embodiment, the terminating molecules R³-OH may be methanol, so that the resulting substituted monomer C is H₃COOC-R²-COOCH₃.

**Condensation monomer:** A short condensation product from both individual monomers A and B, with the formula A'-B"-A', wherein A' and B" have the below definitions. More specifically, the condensation monomer A'-B"-A' is HO-R¹-OOC-R²-COO-R¹-OH, wherein R¹ and R² are as defined above.

**Reacted monomer:** A building block of the polymer in reacted form in any larger molecule. Possible forms are A', A", B' and B'', with A' being identical to H'-A" ' and B' being identical to H'-B" ' .

According to the present invention
an **A' unit** represents a reacted monomer A which is a diol with 1 reacted end group and one unreacted -OH end group and having the formula HO-R¹-O-;
an **A" unit** represents a reacted monomer A which is a diol with 2 reacted end groups and having the formula -O-R¹-O-,
the **reacted A units** represent the sum of all reacted monomer units A' and A" and
the **total A units** represent the sum of all reacted and unreacted monomer units A, A' and A".

According to the present invention
a **B' unit** represents a reacted monomer B which is a diacid with 1 reacted end group and one unreacted -COOH end group and having the formula HOOC-R²-CO-;
a **B" unit** represents a reacted monomer B with 2 reacted end groups, preferably a diacid with 2 reacted end groups and having the formula -OC-R²-CO-, wherein
the **reacted B units** represent the sum of all reacted monomer units B' and B" and
the **total B units** represent the sum of all reacted and unreacted monomer units B, B' and B".

For example, the total B units is the total amount of B units in a given product and is the molar sum of all molecules containing a B unit multiplied by the number of B units in the respective molecules. For example, a product containing monomers B and A'-B"-A' as well as dimer A'-B"-A"-B"-A' has a total amount of B units = mol B + 1 * mol A'-B"-A' + 2 * mol A'-B"-A"-B"-A'.

The following terms comprise the listed mole amount per substance:

| | Individual monomer A | Individual monomer B | Monomer A' -B' | Monomer A' -B" -A' | Dimer A' -B" -A" -B" -A' |
|---|---|---|---|---|---|
| Individual monomer A HO-R²-O-H | 1 | | | | |
| Individual monomer B HOOC-R²-COOH | | 1 | | | |
| A' units HO-R¹-O- | | | 1 | 2 | 2 |
| A" units -O-R¹-O- | | | | | 1 |
| reacted A units (A' + A") | | | 1 | 2 | 3 |
| Total A units (A + A' + A") | 1 | | 1 | 2 | 3 |
| B' units HOOC-R²-CO- | | | 1 | | |
| B" units -OC-R²-CO- | | | | 1 | 2 |
| reacted B units (B' + B") | | | 1 | 1 | 2 |
| total B units (B + B' + B") | | 1 | 1 | 1 | 2 |

| | | | | | |
|---|---|---|---|---|---|
| **Repeat unit:** A condensation product from one of each individual monomer A and B. Possible forms are -A"-B"- having the formula [-O-R¹-OOC-R²-CO-], which would provide the repeating structure in the middle of a molecule, or A'-B''- and -B"-A', which would provide the structure at the end of a molecule. A single repeat unit is also present in the condensation monomer A'-B'. **Dimer:** A condensation product of both individual monomers with two repeat units. Possible forms are A'-B"-A"-B"-A' and B'-A"-B"-A"-B'. The condensation product B'-A"-B"-A"-B' would be considered as a trimer and therefore part of the oligomers. **Oligomer:** A condensation product of both individual monomers A and B with several repeat units in the general form of E[-A"-B"]ₙ-E, where 3<= n <=9, -A"-B"- has the formula [-O-R¹-OOC-R²-CO-] and E = H', A' or B'. **Condensation Polymer:** A condensation product of both individual monomers with a large number of repeat units in the general form of E[-A"-B"]ₙ-E, where n >9, -A"-B"- has the formula [-O-R¹-OOC-R²-CO-] and E = H', A' or B'. According to the present invention, the condensation polymer is a polyester. | | | | | |

Polyesters are obtained by a polycondensation reaction with elimination of a low molecular weight reaction product. The polycondensation can be effected directly between the monomers. The polycondensation may also be effected via an intermediate product, which is subsequently reacted by transesterification with elimination of a low molecular weight reaction product, or which is reacted by ring-opening polymerization.

The polyesters thus obtained have a substantially linear polymer chain. It is however possible for a small number of branches to form.

Preferred polyesters according to the present invention are thermoplastic polyesters.

Polyesters are polymers which are usually obtained by polycondensation from a diol component having the general formula HO-R¹-OH and a dicarboxylic acid component having the general formula HOOC-R²-COOH, where R¹ and R² are usually aliphatic hydrocarbons containing 1 to 15 carbon atoms, aromatic hydrocarbons containing 1 to 3 aromatic rings, cyclic hydrocarbons containing 4 to 10 carbon atoms or heterocyclic rings containing 1 to 3 oxygen atoms and 3 to 10 carbon atoms. Typically, linear or cyclic diol components and typically linear, aromatic, or heterocyclic dicarboxylic acid components are used. Instead of the dicarboxylic acid it is also possible to use its corresponding di-esters, typically its dimethyl ester. Preferred examples of such polyesters are polyethylene terephthalate (PET) which is typically obtained from terephthalic acid (TPA) and ethylene glycol (EG), polybutylene terephthalate (PBT) obtained from terephthalic acid (TPA) and 1,4-butylene diol (BD), polytrimethylene terephthalate (PTT) obtained from terephthalic acid (TPA) and 1,3-propane diol (PB), polyethylene furanoate (PEF) obtained from 2,5-furandicarboxylic acid (FDCA) and ethylene glycol (EG), polytrimethylene furanoate (PTF) obtained from 2,5-furandicarboxylic acid (FDCA) and propane diol (PB), polybutylene succinate (PBS) obtained from succinic acid (SA) and butane 1,4-diol (BD), polybutylene adipate (PBA) obtained from adipic acid (AdA) and 1,4-butane diol (BD), polybutylene adipate-terephthalate (PBAT) obtained from adipic acid (AdA) terephthalic acid (TPA) and 1,4-butane diol (BD) and polyethylene naphthalate (PEN) obtained from naphthalene-2,6-dicarboxylic acid and ethylene glycol, where all of the above polyesters may be present in the form of a homopolymer or as copolymers.

The present invention describes a method to prepare polyesters from their monomers, whereas at least one monomer is a recycled monomer product obtained from a polyester. The polyester prepared from said recycled monomer product and the polyester providing the source for the recycled monomer product may be identical or may differ in composition. The differences in composition may result from the use of different monomers and/or from the use of different amounts of common monomers when preparing said polyester. The differences in composition may also result from blends of different polyesters. In accordance with the present invention, at least one common monomer is present in the polyester providing the source of the recycled monomer product, and the polyester prepared from said recycled monomer product.

For example, PET may be depolymerized using butane diol as a reaction partner, and the resulting recycled monomer product may be re-polymerized to form PBT. Alternatively, a PET copolymer with a high amount of diol comonomers may be depolymerized using ethylene glycol as a reaction partner, and the resulting recycled monomer product may be re-polymerized to form PET with a lower comonomer content.

Polyesters according to the present invention have a general structure comprising repeat units [A"-B"-] specifically having the formula [-O-R¹-OOC-R²-CO-]. These polyesters can be recycled by depolymerization with the addition of a diol monomer A having the formula HO-R¹-OH. The depolymerization results in a recycled monomer product comprising a condensation monomer, which as defined above is in the form of A'-B"-A' and specifically has the formula HO-R¹-OOC-R²-COO-R¹-OH.

The present invention describes a process to prepare polyesters from their monomers, whereas at least one monomer is a modified recycled monomer product obtained from a polyester. The modified recycled monomer product is obtained by reacting recycled monomer with water. As described above the recycled monomer product contains a condensation monomer in the form of A'-B"-A' and specifically has the formula HO-R¹-OOC-R²-COO-R¹-OH. The resulting modified recycled monomer product contains a modified condensation monomer in the form of A'-B' and specifically has the formula HO-R¹-OOC-R²-COOH.

According to a preferred embodiment, the polyester is polyethylene terephthalate (PET) or a copolymer thereof. It is preferred that depolymerization is carried out with ethylene glycol as diol monomer A. In this case, the recycled monomer product comprises as condensation monomer BHET and as modified condensation monomer mono(2-hydroxyethyl) terephthalate (MHET).

The depolymerization can lead to various side products, including a side product with the general structure A'-B' specifically having the formula HO-R¹-OOC-R²-COOH, or diacid monomer B having the formula HOOC-R²-COOH, and partial depolymerization products, including dimers as well as oligomers. Other variants of the above side products containing comonomers can be formed as well.

Preferably, the recycled monomer product comprises at least 50% by weight of said condensation monomer in the form of A'-B"-A' and specifically having the formula HO-R¹-OOC-R²-COO-R¹-OH. Preferably, the recycled monomer product comprises at least 50%, more preferably at least 70%, most preferably at least 80% on dry basis of said condensation monomer, whereas the remainder may be in the form of dimers, oligomers and other side products. In one preferred embodiment the dry recycled monomer product comprises less than 90% on dry basis of said condensation monomer. As used herein, dry basis refers to the total solid content in the recycled monomer product without any liquids such as residual diol, water or inert solvents.

This is sufficient to obtain good quality recycled monomer products and at the same time reduces the amount of yield loss due to removal of oligomers and dimers.

In general, all monomer products containing more than 50% by weight of recycled monomer units are considered as recycled monomer products. Water or inert solvents which may optionally be present in the monomer product are not considered for the calculation.

Inert solvents are any liquids which dissolve or disperse at least a portion of a solid substance and do not or at least only to a small degree react with the solid substance at the temperature where the solution or dispersion is formed.

Preferably, monomer products containing more than 70% by weight of recycled monomer units are used in the method of the present invention.

Typically, the condensation monomer is solid at ambient conditions. Ambient conditions refer to normal temperature and pressure (293.15 K, absolute pressure 101.325 kPa). Typical condensation monomers have a melting point above 50°C. More typical is a melting point above 70°C. The melting point is the temperature where the pure condensation monomer melts. The actual temperature for melting the condensation monomer may be lower, as for example the presence of substances which are liquid at ambient conditions may reduce the melting temperature.

According to the present invention, the modified recycled monomer product obtained in step b1) of the process of the present invention described above is used for the polymerization of a polyester in step b2) of the process of the present invention.

In a preferred embodiment of the present invention, after step b1) the pressure is reduced to remove a vapor phase containing water and diol monomer A. This removal of vapor phase may occur in several steps and may be aided by a further temperature increase. In a preferred embodiment, the pressure is released to obtain ambient pressure. In an optionally preferred embodiment, the pressure is reduced to a pressure below ambient pressure. A preferred pressure reduction reaches 0.5 to 0.05 bar absolute pressure.

In general, as more condensation monomer is converted by water in step b1), more diol monomer A is obtained. Considering that diol monomer A typically has a higher boiling point than water, higher temperatures and/or lower pressure are required to reduce the liquid content. If the recycled monomer product will be converted to solid particles, the content of residual liquid, in this case monomer diol A and water, must be low enough for the particles to be suitable for bulk material handling. Therefore, the solid particles preferably contain less than 10% by weight, more preferably less than 6% by weight, of a substance which is liquid at normal conditions. The percentage is calculated as a ratio of the liquid to the particle weight including the liquid.

The process to prepare the modified recycled monomer product in step b1) may be a process step preceding a slurry preparation step of a melt phase polymerization plant.

If the modified recycled monomer product obtained in step b1) is directly used for subsequent polymerization in step b2), step b1) may be a process step incorporated into a melt phase polymerization plant. In a preferred embodiment of the present invention, the reactor to which the recycled monomer product and the water are provided for carrying out step b1) is incorporated into a melt phase polymerization plant having previously been used to prepare a polyester from a diol monomer A and a diacid monomer B. Preferably, the reactor to which the recycled monomer product and the water are provided has previously been used for esterification of a diol monomer A and a diacid monomer B, commonly named an esterification reactor. Such an esterification reactor may be a stand-alone vessel or a section in a vessel that combines esterification with further processing steps. If a split of primary esterification and secondary esterification provides two esterification reactors, preferably the reactor for primary esterification is used.

In this case, a polyester with a COOH end group concentration above 10 mol-% is produced by a process comprising the following steps:
- providing a recycled monomer product with less than 10 mol-% of COOH end groups, obtained by steps a1) to a3) of the process of the present invention, to an esterification reactor;
- providing water to the esterification reactor;
- thereafter performing step b1) of the process of the present invention by heating and exposing the recycled monomer product in the esterification reactor to an elevated pressure between 0.5 and 10 bar above atmospheric pressure in the presence of the provided water at a temperature between 120°C and 170°C;

- thereafter reducing the pressure to allow evaporation of water and diol monomer A;
- thereafter further reducing the pressure and increasing the temperature to conditions suitable for polymerization, thus carrying out step b2) of the process of the present invention,
wherein the water is provided to the esterification reactor in an amount that equals 3 to 50% by weight of the water content in the total of recycled monomer product plus water.

The water in the above embodiment is supplied from a source outside of the reactors used to produce the polyester. It may be supplied in the form of wet condensation monomer, or it may be supplied as a separate stream. The provided water amount does not encompass water which may result from reactions such as water resulting from a reaction of a diol monomer A with a diacid monomer B.

According to a preferred embodiment of the present invention, in step b2) of the process of the present invention the modified recycled monomer product is used to prepare a polyester with a COOH end group concentration of at least 10 mol-%, preferably at least 15 mol-%, more preferably at least 20 mol-%, but not more than 45 mol-%. The preferred polyester has an intrinsic viscosity in the range of 0.4 to 0.9 dl/g, preferably above 0.5 dl/g, more preferably above 0.55 dl/g and preferably below 0.8 dl/g, more preferably below 0.75 dl/g.

The preparation of polyesters has been well described. Various methods for the preparation of polyesters are described in "Modern Polyesters; Wiley Series in polymer Science; 2003; Edited by John Scheirs" (typical examples in chapters 2, 8, 9 and 11).

Typical preparation processes comprise the direct reaction of equal amounts of individual monomers A and B, leading to polyesters with the repeat unit structure [A"-B"-]ₙ specifically having the formula [-O-R¹-OOC-R²-CO-]ₙ which may be terminated by either A' (HO-R²-O-) and/or B' units (-OC-R²-COOH). During the reaction, low molecular weight reaction products may form which have to be removed by evaporation.

Other, so called two step, processes use individual monomer B (HOOC-R²-COOH) and an excess of individual monomer A (HO-R¹-OH) in a first step to form a prepolymer with the repeat unit structure [A"-B"-]ₙ specifically having the formula [-O-R²-OOC-R²-CO-]ₙ, where n is a small number of preferably less than 20 and which is mostly terminated by A' units (HO-R²-O-). During the reaction, low molecular weight reaction products may form, which have to be removed by evaporation.

In a second step, the prepolymer continues to react leading to polyesters with the repeat unit structure [A"-B"-]ₙ specifically having the formula [-O-R²-OOC-R²-CO-]ₙ, which may be terminated by either A' (HO-R¹-O-) and/or B' units(-OC-R²-COOH). Excess individual monomer A (HO-R¹-OH) is removed by evaporation during the second stage.

An alternative option of the two step processes uses substituted monomer C and an excess of individual monomer A (HO-R¹-OH) to form a prepolymer the repeat unit structure [A"-B"-]ₙ specifically having the formula [-O-R¹OOC-R²CO-]ₙ, where n is a small number of preferably less than 20 and which is mostly terminated by A' units (HO-R¹-O-). During the reaction, low molecular weight reaction products may form which have to be removed by evaporation. In a second step, the prepolymer continues to react leading to polyesters with the repeat unit structure [A"-B"-]ₙ specifically having the formula [-O-R¹-OOC-R²-CO-]ₙ, which may be terminated by either A' (HO-R¹-O-) and/or B' units (-OC-R²-COOH). Excess individual monomer A (HO-R¹-OH) is removed by evaporation during the second stage.

Many polyesters have at least one individual monomer A or B which is a liquid at ambient conditions. Typically, the diol, individual monomer A (HO-R¹-OH), is a liquid. This allows to feed the individual monomers as a liquid to the polyester polymerization. If one monomer is liquid and a second monomer is solid, the solid monomer may be dispersed in the liquid monomer, forming a slurry of individual monomers, which is fed to the polyester polymerization.

A conventional plant designed to prepare a polyester from its individual monomers A and B therefore comprises a unit configured to prepare a slurry from individual monomers A and B. This slurry preparation unit has associated supply units which allow providing individual monomer A in liquid form and individual monomer B in solid form. The resulting slurry is then fed to the subsequent step of polyester polymerization.

A conventional plant designed to prepare a polyester from its individual monomers A and B further comprises a unit configured to promote esterification of the supplied monomers A and B. This esterification unit is typically operated at ambient or elevated pressure and allows the release of water which is formed as a result of the esterification reaction. The unit has associated supply units which allow providing the monomers in liquid form, typically as a slurry. The resulting reaction product, typically low molecular weight oligomers, is then fed to the subsequent step of polyester polymerization.

According to the present invention, the recycled monomer product is obtained by depolymerization of a polyester according to the steps a1) to a3) of the process according to the present invention.

In a preferred embodiment of the present invention, before step b1) of the process of the present invention is carried out, the following steps are additionally performed:
a5) cooling the solution to a temperature to cause precipitation of the recycled monomer product including the condensation monomer, and
a6) recovering the precipitated recycled monomer product from the solution.

In a further preferred embodiment of the present invention, between step a3) and step a5) the following additional step is performed:
a4) mixing the depolymerization solution from step a3) with water or an inert solvent to obtain a diluted solution.

According to said preferred embodiment, the recycled monomer product obtained in step a6) contains water and is provided to step b1) .

The waste stream of step a1) of the process of the present invention may be any stream which predominantly comprises polyester. It may further contain contaminants. The contaminants include solid substances which are mixed with the polyester particles. Typical are metal, paper or foreign plastic. The contaminants include solid or liquid substances adhering to the surface of the polyester particles. Typical are residues from a prior use, like sugar or edible oil, or from cross contamination during collection sorting and pre-cleaning, like sand, stones or washing chemicals. The contaminants further include substances inside the polyester particles. Typical are absorbed volatile substances from previous use, like aroma substances, household chemicals or solvents. Typical are also mixed contaminants, like additives such as colorants, inorganic fillers or organic additive carriers. Typical are also mixed contaminants, like different polymers in polymer blends or blends with natural fibers such as cotton. The contaminants also include water and organic substances which adhere to the surface of the waste particles or which are absorbed by the waste particles. Such liquids may be removed by a pre-drying step. It is however preferred to avoid such a drying step and provide the waste stream including the liquid.

The waste stream may result from post-industrial or post-consumer waste. Such waste may be in the form of articles, like containers or fabrics, or it may be in the form of intermediate products like sheets, fiber rolls or preforms for containers, or it may be in the form of raw materials like pellets or shredded solidified melt. The waste stream may have undergone pre-cleaning steps like optical sorting or separation by mechanical means such as density separation or screening to remove foreign polymers, and non-polymeric substances.

The waste stream may have undergone pre-cleaning steps like washing with hot or cold water optionally containing caustic substances and/or detergents to remove surface contaminants.

The waste stream may have undergone pre-cleaning steps like drying at elevated temperatures to remove moisture and/or volatile organic contaminants.

The waste stream may be cut into particles or it may be in the form of dust or powder. Typical particle sizes are in the range of 0.1mm up to several centimeters. For ease of handling particles above 1mm are preferred and to prevent none dissolved particles to block pipes a size below 5cm especially below 3cm is preferred.

The waste stream may comprise liquifying contaminant. Liquifying contaminant is defined as substances which are solid at room temperature but liquid and not soluble in the depolymerization solution formed in step a3) under the conditions of step a3). Liquifying contaminant may be present at a concentration of 0.1% to 48% by weight, based on the entire amount of the waste stream. The meaning of not soluble refers to any part of a substance which is, given its amount, not dissolved. Minor portions which go into solution are not considered.

The waste stream may comprise solid contaminant. Solid contaminant is defined as substances which are solid and not soluble in the depolymerization solution formed in step a3) under the conditions of step a3). Solid contaminant may be present at a concentration of 0.1% to 48% by weight, based on the entire amount of the waste stream. The meaning of not soluble refers to any part of a substance which is, given its amount, not dissolved. Minor portions which go into solution are not considered. According to the present invention the overall content of polyester is above 50%. The use of the preferred embodiment is most suitable if the content of liquifying plus solid contaminant is at a concentration above 1%, more preferred above 2% and most preferably above 5%.

Waste streams with high amounts of liquifying contaminant are for example polyester sheet with a polyolefin layer or a silicone release layer or polyester containers with polyolefin-based barrier layer such as EVOH.

Waste streams with high amounts of solid contaminant are for example polyester fibers mixed with non-melting fiber material like cotton. Other sources of polyester waste streams with high contaminant levels are mixed fraction which may result from cleaning steps in polymer sorting or washing facilities.

Forming the blend in step a2) comprises any form of mixing and mixing devices that can be used to mix a typically solid or liquid waste stream and a typically liquid diol monomer. Alternatively, it comprises any form of mixing and mixing devices that can be used to mix a typically solid waste stream and a solid diol monomer. In both cases heating may be applied to facilitate the formation of the blend. Typically, the waste stream is supplied to the mixing device in a controlled amount. This may include any gravimetric or volumetric dosing device, such as loss in weight feeders, belt scales or an air lock. The supply of the waste stream in solid form may comprise pneumatic conveying, screw conveying, belt conveying and/or gravimetric feed. An extruder with or without a dosing pump and or a melt filter may be used to supply the waste stream in liquid form. In this case it is preferable to supply the liquid waste stream at a position to the mixing device which is below the fill level of the waste stream to promote immediate contact between the waste stream and the diol. Preferably the supply of the liquid waste stream occurs through multiple openings with provides a large surface contact area between the waste stream and the diol monomer. This is especially important if the melting point of the liquid waste stream is above the boiling temperature of the diol monomer.

According to the present invention at least one diol monomer HO-R1-OH, which is capable of reacting with the polyester is used. Typically, only one diol monomer is used. The use of different diol monomers will result in a mixed recycled depolymerization product, which may only be useful for special co-polyesters. Nevertheless, any diol monomer may contain small amounts of impurities, like dimers thereof. Typically, the diol monomer is supplied to the mixing device in a controlled amount. This may include any gravimetric or volumetric dosing device. Alternatively, a weight or volume difference from a supply tank is used. Typically, the diol monomer is supplied in liquid form through a pump.

The ratio of the solid waste stream and the diol monomer has a significant impact on the final composition of the recycled monomer product after depolymerization but also the economics of the recycling process. In a preferred embodiment the ratio of the sum of all diol monomers added in step a2) to the polyester repeat units contained in the waste stream from step a1) is in the range between 5 to 1 and 15 to 1, wherein the ratio is a molar ratio.

The blend may further comprise a catalyst to facilitate the subsequent depolymerization reaction. Such a catalyst may be in the form of a metal salt or in the form of an organic substance. Also, organic metal complexes can be used. The catalyst may be added in solid or liquid form as a pure substance or in solution. When a solution is used the diol monomer is the most suitable solvent. Forming the blend may be accomplished in continuous fashion or by preparing individual batches.

One option is to use an agitated vessel to form the blend. Depending on the ratio of liquid to solid and the bulk density of the solids the solids may not be fully submerged in the liquid. In that case forming the blend may require some depolymerization and partial dissolution of solids in the liquid before a homogeneous blend is formed. In that case step a2) and a3) overlap. The steps a2) and a3) also overlap in a continuous process where diol monomer and the waste stream are supplied to depolymerization reactor in which depolymerization is already ongoing. Such a procedure is explained in US-4,609,680.

Another option is to use an extruder to melt the polyester and mix it with a diol monomer.

Step a3) comprises the depolymerization of the blend obtained in step a2). The conditions suitable for depolymerization of the polyester in the waste stream are well known in the industry. As an example, various methods for the depolymerization of PET are described in "Recycling of Polyethylene Terephthalate Bottles; Elsevier; 1st ed. 2018, Edited by Sabu Thomas". According to the present invention, depolymerization conditions must be sufficient to obtain a monomer product which predominantly consists of a condensation monomer with the structure A'-B"-A' having the formula HO-R¹-OOC-R²-COO-R¹-OH.

As indicated before, the mixing of step a2) and the depolymerization of step a3) may overlap as depolymerization may already start during mixing. In one preferred embodiment, hot diol monomer is mixed with the waste stream to preheat the waste stream to a temperature sufficient to initiate depolymerization. A suitable temperature is typically above 130°C and at least 20°C below the boiling temperature of the diol monomer. In any case the final level of depolymerization is obtained in step a3). Depolymerization occurs in a reaction vessel. One option is to use an agitated vessel for depolymerization. In a batch process, this may be the same vessel as has been used in step a2). In a continuous process, a separate agitated vessel or a series of agitated vessels is preferred. Alternatively, a pipe reactor may be used instead of one or several of the agitated vessels. Typically, the reaction vessel comprises heating means, such as mantle heating, internal heating coils or an external heat exchanger through which a part of the depolymerization solution is pumped and returned to the vessel.

The vessel is heated to conditions suitable for the depolymerization of the polyester. Typically, a temperature of 170°C has to be exceeded to reach sufficient depolymerization. Preferably the temperature is raised up to the boiling temperature of the diol monomer. The diol monomer may contain other liquids. As a consequence, the boiling temperature may be below the boiling temperature of the diol monomer. As the other liquids evaporate from the depolymerization solution, the boiling temperature increases. As recycled monomer product is formed and dissolved in the diol monomer, the boiling temperature of the diol monomer increases above its initial boiling temperature. Preferably sufficient heating is provided to maintain boiling. The reaction vessel is typically operated with a pressure range of 500mbar to 5bar. It is however preferred to operate the reaction vessel at approximately atmospheric pressure in the range of 1bar ± 100mbar. Especially preferred is to operate the reaction vessel a slight overpressure, up to 100mbar, to allow the transfer of evaporated liquids to subsequent process steps. The pressures refer to absolute pressure if not noted differently.

In a series of reaction vessels, temperature and pressure can change from one vessel to the next. Especially if an extruder is used in step a2), higher temperatures and pressures may be initially used. However, according to the present invention such an extruded blend would be subsequently transferred to a reactor within the above-mentioned specifications.

A preferred series of reactors uses a first reactor at approximately atmospheric pressure at the boiling point of the solution, a second reactor at an overpressure of up to 5 bar at a temperature higher than the first reactor and a third reactor at a pressure below the second reactor, preferably at approximately atmospheric pressure. This series allows to depolymerize in the first reactor while simultaneously removing volatile contaminants. It then allows to increase depolymerization speed in the second reactor and it allows to evaporate some of the diol monomer from the third reactor.

The depolymerization is continued until a depolymerization solution has formed. Except for some contaminants the waste stream should be fully dissolved in the depolymerization solution. Long reaction times at high temperature should be avoided as this causes the formation of side products, like the formation of dimers of the diol monomer. The residence time for the steps a2) and a3) together is typically in the range of 20 minutes to 6 hours. In cases of step a3) occurring at approximately ambient pressure with boiling diol monomer, residence times in the range of 1 to 5 hours are preferred. In cases where the waste stream is preheated as described above, residence times including such preheating may reach up to 24 hours.

The depolymerization solution contains a recycled monomer product, where the monomer product contains a condensation monomer having the formula HO-R1-OOC-R2-COO-R1-OH.

Depolymerization is carried out under reflux. This is typically done to maintain the ratio of polyester repeat units to diol monomer constant. This means that the majority of organic contaminants remains in the depolymerization solution. According to a preferred embodiment of the present invention, the blend in step a2) contains at least one volatile organic contaminant.

In a preferred embodiment, the present invention provides for a process which allows the removal of volatile organic contaminants directly during the depolymerization process and therefore reducing the contaminant load on subsequent cleaning steps. While such removal for volatile contaminants with a very low boiling point or with very low solubility can be achieved by limiting the reflux effectiveness, it is not possible to remove volatile organic contaminants with high solubility in the diol monomer A at depolymerization conditions in a system which retains the diol monomer A.

It has been found that the removal of organic contaminants can be promoted by evaporation of these contaminants together with diol monomer A, either causing a reduction in diol monomer A to polyester repeat unit ratio, or requiring replacement of evaporated diol monomer A with separately purified diol monomer A or a combination thereof.

Volatile organic contaminants (VOCs) are a group of substances with a low boiling point. There is no clear definition for the term volatile. In the context of the present invention the term is used for organic contaminants with a normal boiling point more than 50°C below the boiling point of the diol monomer A used in the depolymerization step a3). Water is a volatile substance, but not a volatile organic contaminant. Other low boiling diols are volatile organic substances, but not volatile organic contaminants.

According to a preferred embodiment of the present invention, the blend in step a2) contains at least one volatile organic contaminant and at least 15%, but not more than 150% by weight, of the diol monomer A supplied to step a2) are evaporated during the depolymerization step a3). The evaporated diol monomer A may lead to a reduction in the diol monomer A content in the depolymerization step a3). Alternatively, the evaporated diol monomer A or parts thereof may be replaced by diol monomer A of high purity introduced to step a3). Such diol monomer A of high purity cannot be obtained by a simple reflux cooler. It requires a separate purification unit such as distillation column and/or a membrane filtration unit. Preferably the amount of volatile organic contaminant introduced into the depolymerization step a3) as a result of replacing the diol monomer A is below the amount of volatile organic contaminant introduced into the depolymerization step a3) from step a2). An evaporation percentage at or above 100% is only feasible if replacement occurs.

To obtain a low concentration of volatile organic contaminant in the depolymerization solution after step a3) it is preferable to evaporate at least 20%, preferably at least 25%, more preferably at least 30%, by weight of diol monomer A. To limit energy consumption associated with evaporation it is preferable to evaporate not more than 100%, preferably not more than 80%, more preferably not more than 70%, by weight of diol monomer A.

According to a preferred embodiment of the present invention, at least 15%, but not more than 60%, by weight of the diol monomer A are evaporated and removed during the depolymerization step a3). The evaporation occurs due to heating of the diol monomer A and, after depolymerization has been initiated, due to heating of the depolymerization solution to its pressure dependent boiling point.

According to a further preferred embodiment of the present invention, the removal of the diol monomer A is sufficient to increase the boiling point of the depolymerization solution above the pressure dependent boiling point of the diol monomer A by at least 5°C, more preferably by at least 8°C, most preferably by at least 10°C.

The meaning of "removed" provides that the evaporated stream is directed away from a step in which the evaporation occurred. This excludes a liquid return from a reflux cooler. In a batch process the contained mass reduces as a result of the evaporation. In a continuous process the mass balance between incoming and outgoing streams must be in an equilibrium. Nevertheless, "removed" provides that there is no direct feed-back stream from the unit to which the vapor is directed into the unit where the evaporation occurs. The percentages are calculated based on the diol monomer amount initially added in step a2), including the water content in the diol monomer, as weight percent.

It is however important to limit the evaporation in such a way that the mole ratio is not shifted in a way that changes the equilibrium in the depolymerization solution too much towards higher oligomer contents. The ratio of the diol monomers to the polyester repeat units contained in the depolymerization solution should therefore remain above 4 to 1. Given the upper limit and the depolymerization reaction the upper limit decreases to 14 to 1.

In a continuous process in a single reactor, the residual water content remains slightly higher. If a series of continuously operated reactors is used and EG is evaporated sequentially, then a residual water content similar to a batch process can be reached. Evaporation can be initiated simply by heating at a given pressure, by applying a reduced pressure at a given temperature or by compression, followed by heating and decompression, the latter being known as flash evaporation.

In a preferred embodiment, the evaporated liquids are directed to a separation unit, configured in such a way that a stream of recycled diol monomer is obtained and at least a part of the recycled diol monomer is subsequently used in step a2).

The above preferred removal of organic contaminants by evaporation together with diol monomer A can also be favorably used in processes where the produced recycled monomer product is not subsequently converted in a step b1) to a modified recycled monomer product.

Thus, the present invention also provides for a process for the preparation of a polyester with a repeat unit [-A"-B"] having the formula [-O-R¹-OOC-R²-CO-] , comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
b2) heating the recycled monomer product to obtain a polyester polymer,
characterized in that the blend formed in step a2) contains at least one volatile organic contaminant, and at least 15% but not more than 150 % by weight of the diol monomer A provided to step a2) are evaporated during step a3).

In said process, step b1) is omitted.

According to the process of the present invention, the depolymerization solution obtained in step a3) may be directly processed further in step b1) to obtain a modified recycled monomer product.

According to a preferred embodiment of the present invention, before being processed further in step b1), said depolymerization solution obtained in step a3) may be subjected to additional processing steps a4), a5) and a6), which will be discussed below.

In an optional step a4), the depolymerization solution from step a3) is mixed with water or an inert solvent to obtain a diluted solution. Any mixing apparatus suitable to mix two different liquids may be used. In a batch process, this may be the same vessel as has been used in step a3), or it may be a subsequent vessel. In a continuous process, a separate apparatus is preferred. The amount of water or inert solvent compared to the amount of diol monomer is in the range between 5:1 and 1:3. Preferred is a mixing ratio below 3:1. Preferred is a mixing ratio above 1:1.5. The mixing ratio is expressed as volume ratio, based on the diol monomer amount in the depolymerization solution at the beginning of the water addition. Most preferred is a water addition which is equal to or exceeds the amount of diol monomer which was added into step a2).

The mixing may comprise adding all water or inert solvent at one position into the full amount of the depolymerization solution. The mixing may also involve sequentially adding parts of the water or inert solvent at different positions. The mixing may comprise adding all water or inert solvent into only a part of the depolymerization solution and a subsequent mixing with the rest of the depolymerization solution.

Alternatively, the mixing may comprise adding all depolymerization solution at one position into the full amount of water or inert solvent. The mixing may also involve sequentially adding parts of the depolymerization solution or inert solvent at different positions. The mixing may comprise adding only a part of the depolymerization solution into all water or inert solvent and a subsequent mixing with the rest of the depolymerization solution.

Alternatively, the mixing may comprise adding depolymerization to an already premixed diluted depolymerization solution. The required water may be added simultaneously, before or after the addition of the depolymerization solution. Again, the individual liquids may be provided to only one position or to multiple positions.

Alternatively, the mixing may comprise mixing a continuous flow of depolymerization solution with a continuous flow of water or inert solvent.

Alternatively, the mixing may comprise mixing a continuous flow of depolymerization solution with a continuous flow of an already premixed diluted depolymerization solution.

In any case mixing should be performed in a way that a supercooling of the depolymerization solution is avoided to prevent a premature crystallization of condensation monomer, dimer and/or oligomers thereof.

In a preferred embodiment the flows and the temperatures of the two liquid streams should be adjusted before mixing in such a way that without the need for evaporation the resulting diluted solution reaches a temperature below its boiling point but above a temperature at which precipitation of the condensation monomer would start. In case of a depolymerization solution resulting from polyethylene terephthalate and ethylene glycol, a temperature of the diluted solution above 60°C and below 110°C is preferred. More preferred is a temperature above 70°C and below 100°C. During the mixing process some vapor of water or inert solvent may form. Typically, these vapors are condensed and returned to the diluted solution or transferred to a solvent recovery unit.

In general, any shock cooling should be prevented. Nevertheless, it is also necessary to prevent the formation of excessive amounts of a side product with the general structure A'-B' specifically having the formula HO-R¹-OOC-R²-COOH. This requires quick cooling below 90°C. It is therefore preferred that the water addition is performed in such a way that the solution reaches a temperature below 90°C within 1 hour after the beginning of the water addition. Preferably the solution reaches a temperature below 90°C within 30 minutes.

In a preferred embodiment of the present invention the water addition is performed in such a way that the solution is present in an agitated vessel and the water is added in multiple positions into the vessel.

In another a preferred embodiment the water addition is performed in such a way that the solution flows through a pipe and the water is injected into the pipe.

In a preferred embodiment, the diluted solution from step a4) is cooled in step a5). In another preferred embodiment, the depolymerization solution from step a3) is directly cooled in step a5), without carrying out step a4).

Any cooling apparatus suitable to cool a liquid and initiate precipitation of a dissolved solid may be used. Typically, an agitated vessel would be used. Typically, the vessel comprises cooling means, such as mantle cooling, internal cooling coils or an external heat exchanger through which a part of the diluted solution is pumped and returned to the vessel. Alternatively, at least a part of the cooling may occur in a plate or pipe heat exchanger. Cooling must be sufficient to cause precipitation the majority of dissolved monomer product and especially the contained condensation monomer. However, cooling must maintain the diluted solution in liquid form. Therefore, cooling is limited to the melting temperature of the diluted solution. In the presence of water-cooling temperatures between 0°C and 25°C, preferred below 15°C and more preferred below 10°C are used. The cooling time must be long enough to allow to cause precipitation of the majority of dissolved monomer product. As the cooling rate is an important factor in the development of the crystal size of the precipitating solids, longer cooling times are preferred to achieve easy to filter solid particles. Typical cooling times are in the range between 1 to 16 hours. Cooling may occur by a constant temperature ramp, by a stepwise temperature ramp or by a temperature ramp followed by a holding time. It is preferred to use agitation during cooling to prevent the formation of large gel-like blocks. The agitation must be strong enough to reach the entire liquid volume throughout the cooling process. At the same time high friction should be avoided to prevent the breakage of precipitated solids. Typical agitators are bled stirrers or cup stirrers. At least 80%, preferably at least 90% of the precipitated solids (measured by weight) should have a particle size above 20pm.

As precipitated solids with different particles sizes may form, it is advantageous to employ a process step where liquid from a cooling apparatus is directed to a dynamic separation device, which separates the liquid in a first liquid portion and a second liquid portion, wherein the second liquid portion contains a majority of the particles below a critical size and wherein the second liquid portion is directed back to the cooling apparatus, and the first liquid portion is directed towards further processing. The second liquid portion may be combined with the diluted solution from step a4) before entering the cooling apparatus. Preferably, the second liquid portion contains more than 70% of the particles with a particle size below a critical size. Particles with a critical size are below 10 pm, preferably below 20 um and most preferably below 30 pm. The dynamic separation device may be any device that separates particles in a liquid based on their response to gravitational force. Typical devices are for example decanters, centrifuges, hydro cyclones or simple sedimentation tanks. Preferred are dynamic separation devices that allow a separation in less than 5 minutes, like decanters, centrifuges or hydro cyclones.

The advantage of utilizing such a dynamic separation device is not only the return of particles that are small and hard to filter to an earlier stage of the process, but that there is also a simultaneous separation tendency to provide the first liquid portion with a higher solid content than the second liquid portion. As a consequence, the solid to liquid ratio of the liquid entering the dynamic separation device is reduced compared to a set-up without such a return of particles. The reduced solid to liquid ratio is subsequently aiding the separation by particle size.

In a preferred embodiment, the precipitated solids, comprising the recycled monomer product, from step a5) are separated from the solution in step a6) by any method suitable for a mechanical solid liquid separation. This includes filtration, sedimentation or centrifugation. It does not include thermal methods where the solution is fully evaporated. The advantage of a mechanical separation is the removal of contaminants which are soluble in the solution and therefore separated from the precipitated solids. Typical suitable filtration equipment includes, filter presses, belt filters, or rotating drum filters. The separation may be performed in batch or in continuous mode. Where no suitable continuous equipment is available, alternating batch systems will be used. Following the separation two material fractions are obtained, a solid recycled monomer product, containing the condensation monomer, and a liquid solution. The solution includes diol monomer, water and/or inert solvent as well as contaminants, residual catalyst and soluble components from the polyester depolymerization, such as condensation monomer, dimers, oligomers and other side products of the depolymerization.

One option is to direct, after washing, all of the liquid streams or a part of the liquid streams to a separation unit configured in such a way that a stream of recycled diol monomer is obtained.

Several additional cleaning steps may be used in the preparation of the recycled monomer product.

These cleaning steps may be operated in sequence or partially combined. They include:
- evaporation of volatile contaminants from any material stream of any of steps a1) to a6), especially from step a3). This requires directing the vapor away from a step where evaporation takes place. Optionally a part of the vapor is condensed and returned to the process step. The term "material stream" involves all materials that are used, generated or processed in any of steps a1) to a6), including the waste stream of step a1), the blend formed from the waste stream of a1) and an added diol monomer in step a2), the depolymerization solution obtained in step a3) from said blend, the diluted solution obtained in step a4) from mixing the depolymerization solution with water or an inert solvent, as well as the precipitated recycled monomer product obtained in step a5).
- mechanical separation, such as filtration, of solid contaminants from any solution obtained in any of the steps a3) to a5). This may be done in one or in multiple steps. Separation includes filtration, which may comprise a first straining step with a screen size between 0.1 to 3mm and a second fine filtration with a pores size between 0.1µm and 0.1mm.
- mechanical separation, such as filtration, of precipitated oligomer particles from any solution obtained in any of the steps a3) to a5). This is preferably done after the step a4) at a temperature which allows precipitation of oligomers, but allows to maintain the majority of the dimer and the condensation monomer in solution. Filter aids may be used to facilitate the filtration.
- mechanical separation, such as filtration, of precipitated dimer particles from any solution obtained in any of the steps a3) to a5). This is preferably done after the step a4) at a temperature which allows precipitation of dimers, but allows to maintain the majority of the condensation monomer in solution. Filter aids may be used to facilitate the filtration.
- mechanical separation, such as filtration, of precipitated dimer particles from any solution obtained in any of the steps a3) to a5). This is preferably done after the step a4) at a temperature which allows precipitation of dimers, but allows to maintain the majority of the condensation monomer in solution. Filter aids may be used to facilitate the filtration. Such a procedure for the specific separation of a dimer is explained in WO 2021/032821. Preferably the mechanical separation of oligomers and or dimers is limited in such a way that the amount of oligomers and dimers remaining in solution comprise more than 5% and preferably less than 20% by weight on dry basis. This can be achieved by setting the temperature for precipitation high enough to keep some oligomer and dimer in solution. Alternatively, this can be achieved by limiting the mechanical separation, for example by a large enough screen size, allowing small particles to pass.
- removal of colorants and other high boiling organic contaminants by the use of activated carbon and the subsequent separation of the activated carbon from any solution obtained in any of the steps a3) to a5). Active carbon may be mixed in powder or granular form with the solution and subsequently filtered, or it may be used as a packed bed, where the solution is passed through. Filter aids may be used to facilitate the filtration. The cleaning step using active carbon can also be used after redissolution of the recycled monomer product. A procedure for color removal with activated carbon is explained in US-6,642,350 and US-2006/074136.
- removal of metals by the use of ion exchangers and the subsequent separation of the ion exchangers from any solution obtained in any of the steps a3) to a5). Ion exchangers may be mixed in powder or granular form with the solution and subsequently filtered or they may be used as a packed bed, where the solution is passed through. Filter aids may be used to facilitate the filtration. The cleaning step using ion exchangers can also be used after redissolution of the recycled monomer product. An ion exchange procedure is explained in US,6,642,350 and US,6,630,601.
- redissolution of the obtained recycled monomer product in a diol monomer, water of an inert solvent or a mixture thereof, and a subsequent precipitation of the recycled monomer product and mechanical separation from the solution.
- purging and/or washing of the filter cake obtained in step a6) with air, water, individual monomer and/or an inert solvent.

The above-mentioned cleaning steps may be combined. For example, oligomer removal, active carbon removal and/or removal of the ion exchangers may be combined into one separation step.

After completion of the depolymerization and cleaning steps a recycled monomer product is obtained which contains 3% to 50% by weight, preferably more than 5%, more preferably more than 10% and preferably less than 40% by weight, of a substance which is liquid at ambient conditions wherein said substance is selected from the group consisting of a diol with a melting point below 20°C, water and an inert solvent. Preferably, said substance which is liquid at ambient conditions is selected from the group consisting of a diol with a melting point below 20°C, and water.

Diol monomer, water and inert solvent may be collected from any of the above-mentioned steps. Those streams may be in liquid or in gaseous form. The streams typically contain other substances such as contaminants, residual catalyst and soluble components from the polyester depolymerization, such as condensation monomer, dimers, oligomers and other side products of the depolymerization.

It is desirable to recover individual purified liquid streams for reuse in any of the steps a1) to a6) or other cleaning steps. Typical methods for the recovery of the liquids are membrane filtration or distillation.

In a preferred embodiment a liquid stream is obtained from one or more of the steps including and after step a5), wherein the liquid stream comprises water and diol monomer, and wherein the liquid stream is directed to a separation unit configured in such a way that a stream of recycled diol monomer is obtained and at least a part of the recycled diol monomer is subsequently used in step a2).

In a preferred embodiment, a vapor stream is obtained during step a3), and a liquid stream is obtained from one or more of the steps including and after step a5). Both streams comprise water and diol monomer, and both streams are directed to a common separation unit configured in such a way that a stream of recycled diol monomer is obtained.

The common separation unit preferably comprises at least a distillation column, which allows for a heat exchange between the vapor stream and the liquid stream.

The separation unit may be further configured to recover other liquids, especially water, into individual purified liquid streams.

Depolymerization followed by crystallization of recycled monomer product leads to precipitated solids suspended in solution. The precipitated solids, comprising the recycled monomer product, are separated from the solution by any method suitable for a mechanical solid liquid separation. However, after step a6), some residual solution remains in the recycled monomer product, which is considered a wet solid. Due to the unwanted substances in the liquid it is desirable to remove the liquid by mechanical means, leading to a displacement of liquid in the wet solid. Washing with clean solution or another liquid is known in the state of the art to provide such displacement. However, displacement is incomplete and unwanted substances tend to adhere to the surface of the solids. Using multiple washing steps further reduce the amount of unwanted substances but also increases the amount of washing liquid, which eventually has to be recovered, and increases the loss of recycled monomer product. Also, unwanted substances may be entrapped between multiple solid particles or in voids inside solid particles, which is little affected by additional washing steps.

The liquid content in the wet solid is typically in the range of between 50% and 200%, based on the dry solid weight. Due to the unwanted substances in the liquid it is desirable to remove the liquid by mechanical means, leading to a displacement of liquid in the wet solid. Again, a thermal separation by evaporation would result in dissolved substances remaining in the recycled monomer product, whereas displacement removes such substances together with the solution. This separation may include technologies for purging with an air flow, liquid removal by compression or liquid exchange due to washing.

It has been found that the removal of unwanted substances can be improved if higher temperature washing liquids are used, while the losses can be limited by reusing such washing liquid in preceding process steps.

According to a preferred embodiment of the present invention, washing for displacement is combined with washing for redissolution. Such technologies may be combined and repeated in multiple sequences. It is especially desirable to wash the solids multiple times, followed by at least one purging stage. As a washing liquid a diol monomer, water or an inert solvent may be used.

In a preferred embodiment, the inlet temperature of the washing liquid in a first washing step is below the inlet temperature of the washing liquid in a subsequent washing step. Preferably, at least one subsequent washing step is performed at a washing liquid inlet temperature of 10°C, more preferably 15°C, most preferably 20°C or more above the inlet temperature of the washing liquid in a first washing step. Optionally the washing liquid for at least one subsequent washing step is performed with a washing liquid vapor, such as for example water steam, which condenses when contacting the recycled monomer product. Increasing the washing liquid temperature will increase the solubility of contaminants and optionally residual catalyst. Therefore, the removal of contaminants and optionally residual catalyst is improved. The removal of contaminants and optionally residual catalyst is further improved as the higher solubility of the recycled monomer product causes some of the recycled monomer product to redissolve in the washing solution and therefore to liberate entrapped contaminants or residual catalyst.

The washing step with the higher inlet temperature leads to a filtrate with a higher outlet temperature.

In a preferred embodiment, the temperature of the washing liquid in a first washing step is near the temperature of the recycled monomer product after filtration. Preferably, the temperature of the washing liquid in a first washing step is not more than 10°C, more preferably not more than 5°C above or below the temperature of the recycled monomer product after filtration. Although it would be possible to use washing liquid substantially above the temperature of the recycled monomer product, it is preferable to use the first washing step as a displacement step with minimal redissolution for the highly contaminated residual solution. This way overall redissolution can be reduced to a minimum.

It is preferred to limit the overall use of fresh washing liquid to limit the amount of recycled monomer product being redissolved. As each subsequent washing stage creates a filtrate of higher purity, the filtrate from one stage may be used in a previous washing stage as washing liquid.

According to the present invention the washing liquid from a subsequent washing step has a higher inlet and outlet temperature and therefore results in a filtrate with more dissolved recycled monomer product.

When reusing this filtrate as a washing liquid, cooling by at least 10°C is required, which causes precipitation of recycled monomer product. At least a part of this cooling will already occur as a result of the contact with the recycled monomer product. This way heat from the washing liquid is transferred to the solids, which reduces the overall energy consumption of the process.

Further cooling conditions should be chosen, preferably not more than 1°C/min, to allow sufficient crystal growth and separation of those crystals in the earlier washing step without blocking the flow of washing liquid.

Alternatively, this filtrate may be reused in a process step before separation in step a6). One preferred use is for dilution of the depolymerization solution in step a4). Also, in this case the solution will undergo some cooling as a result of the contact with the recycled monomer product and allow energy transfer to the solids.

The advantage of both return options is that additional dissolved recycled monomer product will be returned to an earlier step.

Overall, the amount of washing liquid should be in the range of 0.1 to 10 times the amount of recycled monomer product. Preferred is an amount below 5 times, especially preferred below 3 times, the amount of recycled monomer product. Preferred is an amount above 0.3 times, especially preferred below 0.5 times, the amount of recycled monomer product.

It is further preferred to perform washing on a thin filter cake having a thickness in the range of 2 to 10 mm, preferably below 8 mm.

Given the low amount of washing liquid per cycle and the thin filter cake, a good distribution of the washing liquid is required. This is preferably achieved by a spray of washing liquid. Preferably the washing liquid is distributed across the filter cake at a flow rate of 0.5 to 5kg/m².

In a preferred embodiment, the following sequence is used to clean the obtained wet solid:
- the wet solid of the recycled monomer product is placed on a filtration device at a temperature TF1;
- washing of recycled monomer product is performed that comprises a sequence of a washing step with water and/or diol monomer A at a temperature TW1 = TF1 +/-10°C, and a washing step with water and/or diol monomer A at a temperature TW2 >= TW1 +10°C;
- optionally at least one air purging step is used after any of the washing steps;
- optionally additional washing steps with water and/or diol monomer A are used;
- optionally at least two washing steps are subsequently carried out in such a way that fresh washing liquid is used in a step which is not the first washing step and filtrate from that washing step is used as a washing solution in a previous washing step, if necessary by reducing its temperature.

Optionally filtrate from one washing step, preferably the filtrate from the washing step that was carried out at a temperature TW2, is used in step preceding the filtration of the recycled monomer product, preferably in the step a4) of diluting depolymerization solution.

The above preferred removal by washing in step a6) can also be favorably used in processes where the produced recycled monomer product is not subsequently converted in a step b1) to a modified recycled monomer product.

Thus, the present invention also provides for a process for the preparation of a polyester with a repeat unit [-A"-B"] having the formula [-O-R¹-OOC-R²-CO-], comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
a5) cooling the solution to a temperature to cause precipitation of the recycled monomer product including the condensation monomer, and
a6) recovering the precipitated recycled monomer product from the solution,
b2) heating the recycled monomer product to obtain a polyester polymer,
characterized in that the recycled monomer product of step a6) contains residual solution in which unwanted substances are present, wherein washing for displacement of residual solution and washing for partial redissolution of recycled monomer product are carried out in at least two subsequent washing steps.

A preferred embodiment of the present invention requires that monomer A (HO-R¹-OH) comprises at least 90% of ethylene glycol (EG), wherein the percentage is calculated as mole percent of the total amount of individual monomer A, and monomer B (HOOC-R²-COOH) comprises at least 90% of terephthalic acid (TPA), wherein the percentage is calculated as mole percent of the total amount of individual monomer B.

Other suitable forms of monomers may comprise any comonomers typically used in manufacturing polyesters, like isophthalic acid (IPA), cyclohexanedimethanol (CHDM) or diethylene glycol (DEG) .

The polyesters made from these monomers belong to the group of polyethylene terephthalates and their copolymers (PET) with a dominant repeat unit [TPA-EG-].

When using PET with EG as individual monomer A for depolymerization, the main monomer product will be bis(hydroxyethyl)terephthalate (BHET). It is obvious that in the presence of comonomers, comonomer-substituted BHET may be formed. For example, DEG may replace EG as reacted monomer A' or A". Upon re-polymerization, again PET will be obtained. Based on the present invention the PET is formed by adding TPA to the BHET.

As an alternative, monomer R³-OH, preferably methanol, may be used for depolymerization. Upon reaction with EG, again BHET will be obtained.

### Comparative example 1

Polyethylene terephthalate (PET), a polyester with the repeat unit [-A"-B"], was prepared using pure bis(2-hydrocyethyl) terephthalate (BHET), a condensation monomer with the formula A'-B"-A', wherein monomer A is predominantly ethylene glycol and monomer B is predominantly terephthalic acid. The BHET contained 15 mol/t of COOH end groups, which is equivalent to around 0.3% mono(2-hydroxyethyl) terephthalate (MHET) content.

The PET had an intrinsic viscosity (IV) of 0.6dl/g, corresponding to a molecular weight of 16400g/mol and a total of 122 end groups, and a carboxylic end group (COOH) amount of 10.7mol/t, equal to a COOH concentration of 8.8%.

For solid state polycondensation (SSP), the PET was crystallized at 175°C, preheated to 195°C and treated in a continuous nitrogen flow at 195°C and finally cooled. It took 18.6 hours to reach an IV of 0.75dl/g. The COOH end group amount was reduced to 7.7 mol/t, equal to a COOH concentration of 8.3%. Overall the SSP reaction was based on 20.5% esterification reactions. The resulting SSP speed was 0.0081 dl/g/h.

The intrinsic viscosity is measured in phenol : dichlorobenzene 50:50, according to standard methods.

### Example 1

PET with an IV of 0.6dl/g was prepared using recycled BHET obtained by the process of the present invention. The PET had a carboxylic end group amount of 15.3 mol/t, equal to a COOH concentration of 12.6%.

For solid state polycondensation (SSP), the PET was crystallized at 175°C, preheated to 195°C and treated in a continuous nitrogen flow at 195°C and finally cooled. It took 13.7 hours to reach an IV of 0.75 dl/g. The COOH end group amount was reduced to 10.8 mol/t, equal to a COOH concentration of 11.6%. Overall the SSP reaction was based on 31.2% esterification reactions. The resulting SSP speed was 0.0109dl/g/h.

### Example 2

PET with an IV of 0.6dl/g was prepared using recycled BHET obtained by the process of the present invention. The PET had a carboxylic end group amount of 28.5 mol/t, equal to a COOH concentration of 23.4%.

For solid state polycondensation (SSP), the PET was crystallized at 175°C, preheated to 195°C and treated in a continuous nitrogen flow at 195°C and finally cooled. It took 8.6 hours to reach an IV of 0.75 dl/g. The COOH end group amount was reduced to 20.8mol/t, equal to a COOH concentration of 22.4%. Overall the SSP reaction was based on 52.9% esterification reactions. The resulting SSP speed was 0.0174dl/g/h.

## Claims

1. A process for the preparation of a polyester with a repeat unit [-A"-B"] having the formula [-O-R¹-OOC-R²-CO-], comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
b1) heating the recycled monomer product together with 3 - 50% by weight of water in a reactor to a temperature above the melting point of the condensation monomer at a pressure of at least 0.5 bar, but not more than 10 bar, above ambient pressure, to obtain a modified recycled monomer product, and
b2) heating the modified recycled monomer product from step b1), preferably at a reduced pressure, to obtain a polyester polymer.

2. The process according to claim 1, **characterized in that** the polyester is polyethylene terephthalate (PET), the diol monomer added in step a2) is ethylene glycol, and the condensation monomer obtained in step a3) is BHET.

3. The process according to any one of claims 1 or 2, **characterized in that** before step b1) the following steps are performed:
a5) cooling the solution to a temperature to cause precipitation of the recycled monomer product including the condensation monomer, and
a6) recovering the precipitated recycled monomer product from the solution.

4. The process according to claim 3, **characterized in that** between step a3) and step a5) the following step is performed: a4) mixing the depolymerization solution from step a3) with water or an inert solvent to obtain a diluted solution, wherein the recycled monomer product obtained in step a6) contains water and is provided to step b1).

5. The process according to any one of claims 1 to 4, **characterized in that** that the recycled monomer product provided to step b1) has a COOH end group concentration of less than 10 mol-%, and the modified recycled monomer product obtained in step b1) has a COOH end group concentration which is at least 9 mol-% higher than the COOH end group concentration of the recycled monomer product obtained in step a3).

6. The process according to claim 5, **characterized in that** the modified recycled monomer product obtained in step b1) contains at least 10 mol-% of a modified condensation monomer having a polyester repeat unit [-A"-B"] with an OH and a COOH end group, and having the formula [HO-R¹-OOC-R²-COOH].

7. A process for the preparation of a polyester with a repeat unit [-A"-B"] having the formula [-O-R¹-OOC-R²-CO-], comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
b2) heating the recycled monomer product to obtain a polyester polymer,
**characterized in that** the blend formed in step a2) contains at least one volatile organic contaminant, and at least 150 but not more than 150 % by weight of the diol monomer A provided to step a2) are evaporated during step a3).

8. The process according to claim 7, further comprising steps a5) and a6) as defined in claim 4.

9. The process according to claim 7, **characterized in that** the evaporation of the diol monomer A during step a3) leads to a reduction in the diol monomer A content in the depolymerization step a3), and the evaporated diol monomer A or parts thereof are optionally replaced by diol monomer A of high purity.

10. The process according to any one of claims 7 to 9, **characterized in that** at least 15% but not more than 60% by weight of the diol monomer A provided to step a2) are evaporated and removed during the depolymerization step a3).

11. A process for the preparation of a polyester with a repeat unit [-A"-B"] having the formula [-O-R¹-OOC-R²-CO-], comprising the steps:
a1) providing a waste stream comprising a polyester with the repeat unit [-A"-B"] having the formula [-O-R¹-OOC-R²-CO-], wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms,
a2) forming a blend comprising the waste stream and at least one diol monomer HO-R¹-OH, which is capable of reacting with the polyester,
a3) exposing the blend obtained in step a2) in a reaction vessel to conditions suitable for a depolymerization of the polyester, resulting in a depolymerization solution containing a recycled monomer product, said recycled monomer product containing at least 50% by weight on dry basis of a condensation monomer, having the formula HO-R¹-OOC-R²-COO-R¹-OH,
a5) cooling the solution to a temperature to cause precipitation of the recycled monomer product including the condensation monomer, and
a6) recovering the precipitated recycled monomer product from the solution,
b2) heating the recycled monomer product to obtain a polyester polymer,
**characterized in that** the recycled monomer product of step a6) contains residual solution in which unwanted substances are present, wherein washing for displacement of residual solution and washing for partial redissolution of recycled monomer product are carried out in at least two subsequent washing steps, and the filtrate from one washing step is used in a process step before recovery in step a6).

12. The process according to claim 11, **characterized in that** washing comprises a sequence of washing steps, wherein the inlet temperature of the washing liquid in one washing step is below, preferably 10°C, more preferably 15°C, most preferably 20°C or more below, the inlet temperature of the washing liquid in at least one subsequent washing step.

13. The process according to claim 11 or 12, **characterized in that** between step a3) and step a5) a step a4) of mixing the depolymerization solution from step a3) with water to obtain a diluted solution is carried out, wherein the filtrate from one washing step, preferably from the at least one subsequent washing step, is used in in step a4).

14. Use of a polyester obtained by the process according to any one of claims 1 to 6 as a starting polyester for solid state polycondensation, wherein in said solid state polycondensation a high viscosity polyester with at least 0.1 dl/g higher IV than the starting polyester is prepared, and the solid state polycondensation reaction is based on esterification and transesterification, where the ratio of esterification to the total of esterification and transesterification is higher than 25%.

15. A modified recycled monomer product, obtainable by the process according to any one of claims 1 to 6, and comprising a blend of a condensation monomer having the formula HO-R¹-OOC-R²-COO-R¹-OH and of a modified condensation monomer having the formula [HO-R¹-OOC-R²-COOH] , wherein R¹ and R² are the same or different and are selected from the group consisting of aliphatic hydrocarbons containing 1 to 15 carbon atoms, aromatic hydrocarbons containing 1 to 3 aromatic rings, cyclic hydrocarbons containing 4 to 10 carbon atoms, and heterocyclic rings containing 1 to 3 oxygen atoms and 3 to 10 carbon atoms, **characterized in that** said modified recycled monomer product has a COOH end group concentration in the range from 10 mol-% to 50 mol-%, preferably 15 mol-% to 45 mol-%, and especially preferred 20 mol-% to 40 mol-%.

16. Use of a melt phase polymerization plant having previously been used to prepare a polyester from a diol monomer A and a diacid monomer B, for carrying out step b1) of the process according to any one of claims 1 to 6.
